# EUROPEAN PATENT APPLICATION

(11) **EP 4 800 009 A1**
(43) Date of publication of application: **02.09.2026**
(21) Application number: 25766463.1
(22) Date of filing: 08.07.2025
(51) Int. Cl.: C07D 239/52

(54) **SYNTHESIS METHOD FOR AZOXYSTROBIN**

(30) Priority: 31.12.2024 CN 202411979270
(71) Applicant: INNER MONGOLIA MIRACULOUS CROP SCIENCECO., LTD., Alxa, Inner Mongolia 750300 (CN)
(72) Inventor: YANG, Guozhong, Alxa, Inner Mongolia 750300 (CN); DONG, Haohao, Alxa, Inner Mongolia 750300 (CN); CHENG, Lihua, Alxa, Inner Mongolia 750300 (CN); PENG, Yanli, Alxa, Inner Mongolia 750300 (CN); SHI, Xiaotuo, Alxa, Inner Mongolia 750300 (CN)
(74) Representative: LLR
(86) International application number: PCT/CN2025/107457
(87) International publication number: WO 2026/144087

(57) **Abstract**

The present application discloses a synthesis method for azoxystrobin, comprising a step of: reacting a compound of Formula I with 2-cyanophenol in a water-oil system of an organic solvent and an aqueous solution of trimethylamine to obtain azoxystrobin, wherein a molar ratio of the compound of Formula I to trimethylamine is 1:(0.96-2). The synthesis method of the present application has advantages of high reaction efficiency, no CO₂ generation, avoidance of the risk of entrained overflow, reduced secondary reactions, reduced solid waste emissions, and environmental friendliness. Use of solid sodium carbonate or potassium carbonate is avoided and the material reaction system is an oil-water reaction system. The reaction efficiency is significantly improved and the yield can reach 95% or above. Both efficiency (timeliness) and yield can be taken into account, and continuous reactions can be realized easily, providing technical support for intelligent, unmanned production. Moreover, trimethylamine can be recycled, reducing the consumption of one starting material. Accordingly, complicated treatment of mixed salts and wastewater thereof can be avoided, thereby significantly reducing energy consumption.

## Description

### Cross Reference

The present application claims priority to Chinese Patent Application No. 202411979270.1, filed with the China National Intellectual Property Administration (CNIPA) on December 31, 2024 and entitled "Synthesis Method for Azoxystrobin", which is incorporated herein by reference in its entirety.

### Technical Field

The present application relates to the technical field of pesticides, and in particular, to a synthesis method for azoxystrobin.

### Background Art

Methyl (E)-2-[2-[6-(2-cyanophenoxy)pyrimidin-4-yloxy]phenyl]-3-methoxyacrylate is a highly effective broad-spectrum agricultural fungicide with multiple effects such as uptake and translocation, prevention, protection, and treatment. It demonstrates excellent efficacy against plant diseases such as powdery mildew, rust, glume blotch, downy mildew, and rice blast. Currently reported synthesis processes for this fungicide typically involve a reaction of methyl (E)-2-[2-(6-chloropyrimidin-4-yloxy)phenyl]-3-methoxyacrylate (a compound of Formula I) with 2-cyanophenol and a solid carbonate or a reaction of methyl (E)-2-[2-(6-chloropyrimidin-4-yloxy)phenyl]-3-methoxyacrylate with an alkali metal salt of 2-cyanophenol, in the presence of a nearly anhydrous organic solvent and a catalyst. However, existing reaction routes still face a series of challenges.

For example, Patents No. WO9208703 and No. EP0382375 both describe a reaction route where methyl (E)-2-[2-(6-chloropyrimidin-4-yloxy)phenyl]-3-methoxyacrylate is mixed with o-hydroxybenzonitrile, using potassium carbonate as an acid scavenger, cuprous chloride as a catalyst, and DMF as a solvent, reacting at 120°C. However, this route presents difficulties in post-treatment and crystallization (requiring 3 weeks at room temperature), making it unsuitable for large-scale industrial production. The process route is as follows:

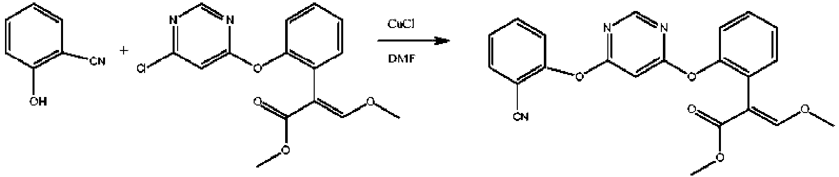

For another example, Patent No. CN101163682B discloses obtaining azoxystrobin by reacting methyl (E)-2-[2-(6-chloropyrimidin-4-yloxy)phenyl]-3-methoxyacrylate with 2-cyanophenol in a DMF slurry using DABCO as a catalyst in the presence of an acid acceptor (potassium carbonate or sodium carbonate), as described in step c) of Example 1, after the reaction was complete, the DMF was removed by vacuum distillation; toluene(160mL) and water(265mL) at 60°C were added to the distillation residues and the two phase mixture was heated to 70-80°C; the mixture was stirred for 40 minutes and then settled and the lower aqueous phase was separated. The reaction mixture in a slurry state underwent vacuum distillation to remove DMF, yielding a distillation residue with a high solid content. This demanded a high-power stirring equipment, and CO₂ as a byproduct was released in the reaction process. Large volumes of solvents and water are added during post-treatment. The water added is used to dissolve the excess acid acceptor (potassium carbonate/sodium carbonate) and the resulting potassium chloride and potassium bicarbonate or sodium chloride and sodium bicarbonate. As can be seen, the post-treatment of the target product is complicated and time-consuming. Hydrochloric acid needs to be added to convert the mixed salts generated to a single salt, during which a significant amount of CO₂ will be released. Saltwater requires heating and distillation for water removal and separation. The whole process involves many devices, high energy consumption, and long steps and is not easy to continue. It is not easy to realize intelligent and continuous industrial production. Many operators are required with high labor costs.

For another example, Patent No. CN109721548B discloses obtaining azoxystrobin by reacting 2-cyanophenol or a salt thereof with methyl (E)-2-[2-(6-chloropyrimidin-4-yloxy)phenyl]-3-methoxyacrylate using trimethylamine as a catalyst in the presence of an acid acceptor (potassium carbonate/sodium carbonate), wherein trimethylamine is used in an amount of 0.5mol% to 15mol% of methyl (E)-2-[2-(6-chloropyrimidin-4-yloxy)phenyl]-3-methoxyacrylate; the acid acceptor is potassium carbonate and/or sodium carbonate; and the reaction typically occurs at 50°C to 120°C for 5 h to 20 h. In Example 1, after the completion of the reaction, 100 g of water was added. The total starting material input for the reaction was 145.5 g. As could be seen, the addition of water was intended to dissolve the excess acid acceptor (potassium carbonate or sodium carbonate) and byproduct salts. Liquid separation yielded an oil phase and a water phase. Although the technical solution for treating the water phase is not specified, it is understood by those skilled in the art that the treatment method would encounter the same unresolved technical problems as described in Patent No. CN101163682B.

In the aforementioned technical solution, the reaction system typically employs 2-cyanophenol or a salt thereof and methyl (E)-2-(2-((6-chloropyrimidin-4-yl)oxy)phenyl)-3-methoxyacrylate as starting materials, with potassium carbonate or sodium carbonate as an acid scavenger (or an acid acceptor). Although this technical solution achieves a relatively high yield of azoxystrobin, the reaction process, involving a solid-liquid mixed system, is dominated by intermittent reactions, and not only requires a solid feeding device, but also faces problems such as release of byproduct CO₂ in the reaction process, long reaction time, low production efficiency, and high energy consumption. In case of continuous operation, not only is complex equipment required, but also the corresponding mass transfer equipment presents high investment costs, leading to increased costs and making it unsuitable for industrial production. Many technicians have tried to enhance the production efficiency for better industrial production. They have made attempts in terms of catalysts and reaction starting materials, but failed to find a method to solve the above-mentioned problems. That is, it is difficult to take both yield and timeliness into account.

The synthesis of azoxystrobin is typically conducted in organic phases. However, carbonates as acid scavengers are basically present in the form of solids in organic solvents (e.g., toluene, DMF) with low solubility, resulting in a solid-liquid heterogeneous slurry reaction system which leads to slow reaction rates with 2-cyanophenol. Even though 2-cyanophenol is fed directly in the form of a potassium salt or a sodium salt, the potassium salt or the sodium salt of 2-cyanophenol also exhibits reduced solubility in an organic solvent, still resulting in a solid-liquid heterogeneous slurry reaction system. Its condensation reaction with methyl (E)-2-(2-((6-chloropyrimidin-4-yl)oxy)phenyl)-3-methoxyacrylate occurs at a low rate, leading to reduced reaction efficiency. As described in existing literature and patents, when sodium carbonate and potassium carbonate are used as acid scavengers, alkaline conditions will be created in the presence of a large amount of water, which, during reaction with heating at a high temperature, may cause easy hydrolysis of methyl (E)-2-(2-((6-chloropyrimidin-4-yl)oxy)phenyl)-3-methoxyacrylate to generate byproducts. As a result, the reaction yield will decrease. Therefore, anhydrous or low-water organic solvent reaction systems are conventionally employed. While there are a series of problems of solid-liquid hybrid reactions, the use of sodium carbonate and potassium carbonate as acid scavengers is still an option to obtain azoxystrobin with a high yield as universally accepted by those skilled in the art. Therefore, many researchers expect to improve the generation efficiency through the selection of catalysts.

Nevertheless, the above technical solutions also have the following problems: costly mass transfer equipment is required in production; a large amount of greenhouse gas CO₂ is released in the reaction process, which requires an addition of an additional tail gas treatment system; and CO₂ overflow is prone to risks of solvent entrainment, foaming, material overflow, and the like. Furthermore, in the reaction process, 2-cyanophenol is prone to cyano-polymerization at a high temperature. Especially under alkaline conditions, both excessive temperature and too long time may result in different degrees of polymerization, and with increasing temperature and prolonging time, the extent of polymerization will increase such that byproducts are increased, leading to reduced yield or purity. When the reaction time is too long, the starting material methyl (E)-2-(2-((6-chloropyrimidin-4-yl)oxy)phenyl)-3-methoxyacrylate will undergo hydrolysis or alcoholysis. In the post-treatment of the reaction slurry, a large amount of water needs to be added to enable liquid separation, and the mixed salts of chloride, bicarbonate, and carbonate solids are present in the water phase. In order to convert the mixed salts into a single chloride, a large amount of hydrochloric acid needs to be added, resulting in high post-treatment costs and increased carbon emissions.

### Summary of the Invention

### Objective of the Invention

In order to overcome the above shortcomings, an objective of the present application is to provide a synthesis method for azoxystrobin featuring high efficiency, resource saving, minimal solid waste generation, low capital input, high degree of automation, continuous operation, low carbon footprint, and high benefits. The inventors have found that using an aqueous solution of trimethylamine as an acid scavenger transforms a material reaction system into an oil-water reaction system, significantly improving the reaction efficiency. Besides, there is no need to use solid potassium carbonate/sodium carbonate, preventing CO₂ gas generation and emission during the reaction process and avoiding the risk of gas-entrained liquid rushing out of the reaction system. The oil-water reaction system is more suitable for use of continuous production equipment, thereby supporting digital, intelligent, and environment-friendly transformation of industrial production while improving competitiveness and achieving higher operational efficiency. Moreover, trimethylamine can be recycled, which circumvents complicated post-treatment processes for mixed salts. Also, liquid separation can be achieved without additional water for dissolving generated chlorides, bicarbonates, and unreacted potassium carbonate/sodium carbonate, thereby reducing water consumption, avoiding treatment of a large volume of mixed salt wastewater, further decreasing energy consumption for industrial production, and lowering industrial production costs.

### Solutions

In order to achieve the objective of the present application, the present application adopts the following technical solutions.

In a first aspect, the present application provides a synthesis method for azoxystrobin, comprising a step of: reacting a compound of Formula I with 2-cyanophenol in a water-oil system of an organic solvent and an aqueous solution of trimethylamine to obtain azoxystrobin, 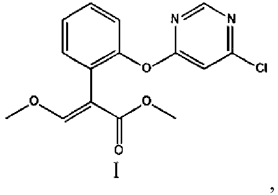 wherein a molar ratio of the compound of Formula I to trimethylamine is 1:(0.96-2).

Further, the molar ratio of the compound of Formula I to trimethylamine is 1:(1-2), optionally 1:(1.06-2), optionally 1:(1.1-2), optionally 1:(1.06-1.8), and optionally 1:(1.1-1.8).

Further, a molar ratio of the compound of Formula I to 2-cyanophenol is 1:(1-5), optionally 1:(1-1.5), and optionally 1:(1-1.2).

Further, a mass fraction of trimethylamine in the aqueous solution of trimethylamine is 20% to 40%, optionally 25% to 40%, optionally 20% to 30%, optionally 25% to 30%, and optionally 30% to 40%;
and/or a molar ratio of the compound of Formula I to water is 1:(4.728-23.64), optionally 1:(4.925-23.64), optionally 1:(5.2205-23.64), optionally 1:(5.66-23.64), optionally 1:(7.65-23.64), optionally 1:(5.66-15.29), and optionally 1:(7.65-15.29);
and/or a molar fraction of water in the water-oil system is 30mol% to 60mol%, optionally 31mol% to 60mol%, optionally 31mol% to 54mol%, optionally 31mol% to 53mol%, and optionally 35mol% to 53mol%.

Further, there is no need to add additional or minimal sodium carbonate or potassium carbonate (note that while there is no need to add additional sodium carbonate or potassium carbonate as an acid scavenger, it should be understood that the addition of minimal sodium carbonate or potassium carbonate to the reaction system has tiny influence on the effects of the present application, can also solve the technical problems to be solved in the present application, and is an equivalent substitution).

Further, an addition amount of the organic solvent is at least an amount for dissolving the compound of Formula I and 2-cyanophenol.

Further, a weight ratio of the compound of Formula I to the organic solvent is 1:(2-8), optionally 1:(2-5.7), optionally 1:(2-4), optionally 1:(2.3-5.7), and optionally 1:(2.3-4).

Further, the organic solvent comprises toluene.

Optionally, the reaction occurs in the water-oil system composed of the organic solvent and the aqueous solution of trimethylamine.

Further, a reaction temperature is 50°C to 170°C, optionally 88°C to 170°C, optionally 97°C to 170°C, optionally 110°C to 170°C, optionally 140°C to 170°C, optionally 140°C to 160°C, optionally 80°C to 150°C, and optionally 80°C to a reflux temperature. Optionally, the reaction temperature is 50°C, 80°C, 88°C, 97°C, 100°C, 110°C, 120°C, 130°C, 140°C, 150°C, 160°C, 170°C, or in ranges between any two of them.

Further, a reaction pressure is greater than normal pressure, optionally 0.12 MPa to 1.2 MPa, optionally 0.29 MPa to 1.2 MPa, optionally 0.5 MPa to 1.2 MPa, and optionally 0.55 MPa to 1.1 MPa.

Further, a reaction time is 5 min to 360 min, optionally 15 min to 360 min, optionally 30 min to 360 min, optionally 5 min to 60 min, optionally 30 min to 60 min, and optionally 5 min to 30 min.

Further, the reaction occurs in a pipe-based continuous reaction equipment; a pipe temperature is 110°C to 170°C, optionally 110°C to 150°C, optionally 140°C to 170°C, optionally 140°C to 160°C, and optionally 140°C to 150°C; and optionally, a reaction pressure is 0.55 MPa to 1.1 MPa, optionally 0.5 MPa to 0.7 MPa.

Further, a kettle-based intermittent reaction or a kettle-based continuous reaction is employed with a reaction temperature of 85°C to 150°C, optionally a reaction temperature of 85°C to 110°C, optionally a reaction temperature of 85°C to 97°C, and optionally a reaction temperature of 85°C to 95°C. Optionally, the reaction temperature is 50°C, 80°C, 88°C, 97°C, 100°C, 110°C, 120°C, 130°C, 140°C, 150°C, or in ranges between any two of them. Optionally, a reaction pressure is normal pressure to 1.1 MPa. Optionally, the reaction pressure is greater than normal pressure, and ≤1.1 MPa. Optionally, the reaction pressure is 0.12 MPa to 1.1 MPa.

The reaction system in the present application can significantly accelerate the reaction by building the pressure and increasing the temperature, and would not produce excess byproducts due to increasing the temperature or building the pressure.

Further, the pipe-based continuous reaction equipment comprises a feeding pump, a safety relief valve, a static mixer, a retention pipe, a back pressure valve, a receiving device, and a heat exchanger.

In a pipe-based continuous reaction, the compound of Formula I, 2-cyanophenol, and the organic solvent are used as a material A, and the aqueous solution of trimethylamine is used as a material B; and a mass ratio of the material A to the material B is (4-7):1 such that a molar ratio of the compound of Formula I to trimethylamine in the system is maintained at 1:(1-2), optionally 1:(1.06-1.8), and optionally 1:(1.1-1.8).

Further, after completion of the reaction, post-treatment is carried out to obtain the azoxystrobin product. Optionally, the post-treatment comprises steps of: direct liquid separation of materials after the completion of the reaction (direct liquid separation refers to no addition of additional water), oil phase desolvation, and purification.

Optionally, the purification comprises redissolution (redissolution may occur in methanol), crystallization, suction filtration, washing and drying.

Further, trimethylamine is recovered from a water phase after liquid separation. Optionally, a recovery process comprises steps of: adjusting a pH of the water phase to 2-8, concentrating, and then adding a base (optionally, the base is used for liberating trimethylamine) to obtain a solution containing trimethylamine.

Further, in the trimethylamine recovery, an addition amount of the base is 0.95 to 1.2 times, optionally 1 to 1.2 times, a molar weight of the compound of Formula I;
and/or in the trimethylamine recovery, the base is an alkali metal hydroxide. Optionally, the alkali metal is sodium or potassium (which may be NaOH or KOH). Other bases may also be used.

### Beneficial Effects

The synthesis method of the present application has advantages of high efficiency, resource saving, minimal solid waste generation, low carbon emissions, and high comprehensive engineering benefits, while reducing capital investment. It enables continuous reactions, improves the reaction efficiency, and provides technical support for continuous and intelligent production, achieving inherently safe design and significantly reducing the number of industrial production workers. According to the present application, the aqueous solution of trimethylamine is used as the acid scavenger, eliminating the need for solid potassium carbonate or sodium carbonate, as well as the conversion of 2-cyanophenol to 2-cyanophenol salts. The material reaction system is an oil-water reaction system, which produces no CO₂ emissions during the reaction process. Moreover, trimethylamine can be recycled, reducing the consumption of one starting material. Accordingly, complicated treatment of mixed salts and the treatment of mixed salt wastewater are avoided. The innovative technical solutions present low energy consumption due to a design from reaction sources. Liquid separation can be realized without an addition of additional water, avoiding wasting of water resources. The oil-water reaction system used in the present application exhibits high mass transfer efficiency and heat transfer efficiency, shortened reaction time, and reduced equipment wear as compared to a solid-liquid slurry reaction system. The present application does not employ potassium carbonate or sodium carbonate, avoiding generation of a large amount of greenhouse gas CO₂ and the risk of overflow (a large amount of greenhouse gas CO₂ may be not conducive to green low-carbon transition, and requires an addition of an additional tail gas treatment system; besides, the solvent would be entrained in emissions, and the reaction mixture is prone to overflow). The use of one starting material is reduced at the solution design stage. Carbon emission reduction and the intrinsic safety of the process are realized innovatively through technological reformation. The synthesis method of the present application can reduce cyano-polymerization of 2-cyanophenol at a high temperature. The reaction time is greatly shortened so that hydrolysis or alcoholysis of the compound of Formula I can be reduced, thereby increasing both yield and purity.

### Brief Description of the Drawings

One or more examples are exemplified by the pictures in the accompanying drawings that correspond thereto and are not intended to be limiting of the examples. As used herein, the word "exemplary" means "serving as an instance or an example, or illustrative". Any example described herein as "exemplary" is not necessarily to be construed as superior to or better than other examples.

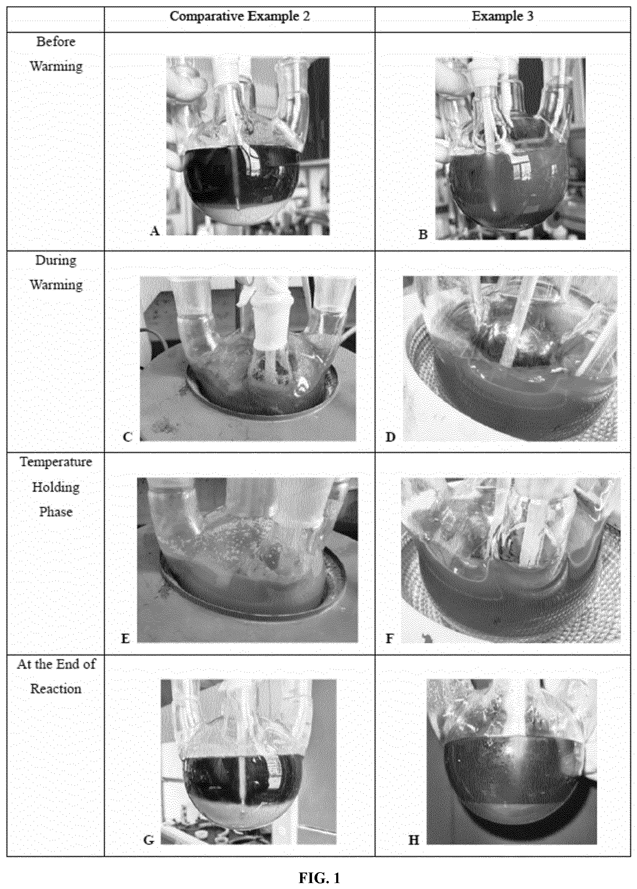

FIG. 1 illustrates changes in material state before and after reactions in Comparative Example 2 and Example 3 of the present application, wherein A shows a material state before warming in Comparative Example 2; C shows a material state during warming in Comparative Example 2; E shows a material state at a temperature holding phase in Comparative Example 2; G shows a material state at the end of reaction in Comparative Example 2; B shows a material state before warming in Example 3; D shows a material state during warming in Example 3; F shows a material state at the temperature holding phase in Example 3; and H shows a material state at the end of reaction in Example 3.
FIG. 2 shows an HPLC chromatogram of an organic phase and detection results after the completion of reaction in Comparative Example 2, wherein 11.528 min is a peak of azoxystrobin, 14 min is a solvent peak, and others are impurity peaks.
FIG. 3 shows an HPLC chromatogram of an organic phase and detection results after the completion of reaction in Example 3, wherein 11.558 min is a peak of azoxystrobin, 14 min is a solvent peak, and others are impurity peaks.

### Detailed Description of the Invention

In order to make the objective, technical solutions, and advantages of the present application clearer, the technical solutions in the examples of the present application are clearly and completely described below with reference to the accompanying drawings in the examples of the present application. Apparently, the described examples are merely a part rather than all of the examples of the present application. All other examples derived from the examples of the present application by a person of ordinary skill in the art without creative efforts shall fall within the protection scope of the present application.

In addition, in order to better explain the present application, a lot of specific details are given in the following particular embodiments. It will be understood by those skilled in the art that the present application may be practiced without certain specific details. In some examples, starting materials, solutions, methods, means, etc., well known to those skilled in the art, are not described in detail so as to highlight the spirit of the present application.

Throughout the specification and claims, the term "comprise" or variations thereof, such as "comprising" or "comprised", will be understood to include the stated components and not to exclude other elements or other components, unless expressly indicated otherwise.

Methyl (E)-2-(2-((6-chloropyrimidin-4-yl)oxy)phenyl)-3-methoxyacrylate, 2-cyanophenol, trimethylamine, and other reagents in the following examples are commercially available. Unless otherwise specified, the reaction processes and results are detected by high-performance liquid chromatography (HPLC), and an external standard method is used for content detection.

A compound of Formula I of the present application, namely methyl (E)-2-(2-((6-chloropyrimidin-4-yl)oxy)phenyl)-3-methoxyacrylate, and 2-cyanophenol (a compound of Formula II) are dissolved in an organic solvent for reacting under the action of an aqueous solution of trimethylamine, generating azoxystrobin (a compound of Formula III). The reaction route is as follows:

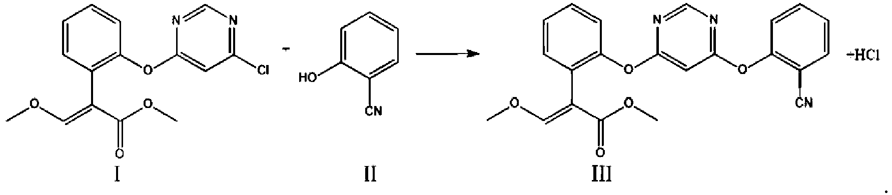

In the following examples, the compound of Formula I refers to methyl (E)-2-(2-((6-chloropyrimidin-4-yl)oxy)phenyl)-3-methoxyacrylate.

In the following examples, a reference for detection of byproducts is as follows: CHEN Hai-yan, TAO Wen-bo, DING Ke-hong. Analysis of Side Reactions in the Synthesis of Azoxystrobin by LC/MS[J]. Pesticides, 2016,55 (10): 725-728. P727 impurity structures 1 and 4 are as follows:

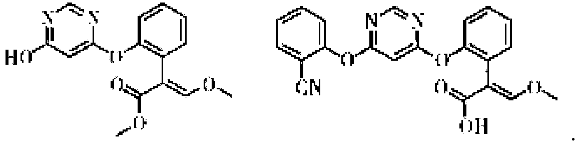

In the following examples, a system pressure refers to a gage pressure (because industrial production records usually use gage pressures, i.e., gage pressures of pressure gages are directly read and recorded, so as to simplify industrial operation by workers and reduce difficulties in production recording) instead of an absolute pressure (absolute pressure = gage pressure + atmospheric pressure). For example, as described in Example 6, the set pressure of the pressure relief valve was 0.55 MPa (i.e., the gage pressure). It is described as the system pressure in some examples (for example, the system pressure in Example 14 was 0.02 MPa, and the absolute pressure (reaction pressure) was 0.12 MPa).

In the following Example 3 and Comparative Example 2, conditions for HPLC of the organic phases after the completion of reactions are as follows: Agilent 1260, chromatographic column: ODS-3 4.6*250 mm 5 µm; a column oven at 30°C; a mobile phase of acetonitrile: water: trifluoroacetic acid = 600:400:2; a detection wavelength of 254 nm; and a flow rate of 1.0 mL/min.

Through continuous research, the inventors have found that using an aqueous solution of trimethylamine as the acid scavenger not only transforms the material reaction system into an oil-water reaction system, significantly improving the reaction efficiency, but also maintains a yield of 95% or above. Both efficiency (timeliness) and yield can be taken into account, and continuous reactions can be realized easily, exhibiting applicability for industrial production. They have found in further research that other tertiary amines such as triethylamine and an aqueous NaOH solution cannot achieve reaction effects similar to those with trimethylamine as the acid scavenger. In particular, some examples are described below.

### Example 1

131 g (0.40 mol) of a compound of Formula I and 51.5 g (0.424 mol) of 2-cyanophenol were added to 301 g of toluene to form a solution, and 83.4 g of an aqueous solution of trimethylamine (30%, 0.424 mol) was added thereto. Refluxing was performed under normal pressure, and the temperature was held for 4.5 h, during which the color of the reaction mixture gradually became lighter. After the completion of temperature holding, the temperature was reduced to 30°C, followed by standing directly, thereby obtaining clear oil and water phases. Both phases were separated. The resulting reddish-brown oil phase was washed, then distilled under reduced pressure to remove toluene, cooled to 60-65°C, added with methanol, warmed for refluxing and dissolution, cooled to around 0°C, crystallized for 2 h, subjected to suction filtration, and washed. The filter cake was dried to obtain 157.6 g of azoxystrobin product with a content of 98.7% and a yield of 96.4%.

### Example 2

131 g (0.40 mol) of a compound of Formula I and 55.8 g (0.46 mol) of 2-cyanophenol were added to 747 g of toluene to form a solution, and 110.1 g of an aqueous solution of trimethylamine (30%, 0.56 mol) was added thereto. Refluxing was performed under normal pressure, and the temperature was held for 5 h, during which the color of the reaction mixture gradually became lighter. After the completion of temperature holding, the temperature was reduced to 30°C, followed by standing directly, thereby obtaining clear oil and water phases. Both phases were separated. The resulting reddish-brown oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 159.2 g of azoxystrobin product with a content of 98.1% and a yield of 96.8%.

### Example 3

131 g (0.40 mol) of a compound of Formula I and 55.8 g (98%, 0.46 mol) of 2-cyanophenol were added to 400 g of toluene to form a solution, and 78.7 g of an aqueous solution of trimethylamine (30%, 0.40 mol) was added thereto. Refluxing was performed under normal pressure and the temperature was held for 6 h, during which the color of the reaction mixture gradually became lighter. After the completion of temperature holding, the temperature was reduced to 30°C, followed by standing directly, thereby obtaining clear oil and water phases. Both phases were separated. The resulting reddish-brown oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 157.7 g of azoxystrobin product with a content of 97.9% and a yield of 95.7%.

### Example 4

131 g (0.40 mol) of a compound of Formula I, 53.4 g (0.44 mol) of 2-cyanophenol, and 500 g of toluene were added to a sealed reaction kettle to form a solution, and after stirring evenly, 102.3 g of an aqueous solution of trimethylamine (30%, 0.52 mol) was added thereto. The reaction kettle was closed, and the temperature was increased to 150°C and held for reacting for 0.5 h, under a system pressure of 0.55 MPa. After the completion of the reaction, the temperature was reduced to around 30°C, followed by standing directly, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 158.4 g of azoxystrobin product with a content of 98.2% and a yield of 96.4%.

### Example 5

131 g (0.40 mol) of a compound of Formula I, 58.3 g (0.48 mol) of 2-cyanophenol, and 500 g of toluene were added to a sealed reaction kettle to form a solution, and after stirring evenly, 118 g of an aqueous solution of trimethylamine (30%, 0.60 mol) was added thereto. The reaction kettle was closed, and the temperature was increased to 160°C and held for reacting for 0.25 h, under a system pressure of 0.77 MPa. After the completion of the reaction, the temperature was reduced to around 30°C, followed by standing directly, thereby obtaining clear oil and water phases. Both phases were separated. The resulting oil phase was washed, then distilled under reduced pressure to remove toluene, cooled to 60°C to 65°C, added with methanol, warmed for refluxing and dissolution, cooled to around -5°C, crystallized for 2 h, subjected to suction filtration, and washed. The filter cake was dried to obtain 158.2 g of azoxystrobin product with a content of 98.0% and a yield of 96.1%.

### Example 6

1310 g (4.00 mol) of a compound of Formula I and 544 g (4.48 mol) of 2-cyanophenol were added to 5246 g of toluene, and stirred until a clear solution was obtained for later use (denoted as material A). 1023 g of an aqueous solution of trimethylamine (30%, 5.20 mol) was weighed for later use (denoted as material B). The material A and the material B were delivered by a metering pump to a static mixer preheated to 150°C through pipes, respectively. An outlet of the static mixer was connected to a sealed reaction kettle. A set pressure of a relief valve at an outlet of the sealed reaction kettle was 0.55 MPa. An outlet of a feed pipe was arranged at the bottom of the reaction kettle, and a discharge port pipe was arranged above the liquid level. A feed mass ratio of the material A to the material B was 6.94:1. A suitable feed flow rate was adjusted. A retention time of the materials in the reaction kettle was controlled to be 0.5 h. After the operation was stable, the product was continuously taken out of the discharge port, cooled, and directly let to stand, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 1585.6 g of azoxystrobin product with a content of 98.3% and a yield of 96.6%.

### Example 7

1310 g (4.00 mol) of a compound of Formula I and 544 g (4.48 mol) of 2-cyanophenol were added to 5240 g of toluene, and stirred until a clear solution was obtained for later use (denoted as material A). 1180 g of an aqueous solution of trimethylamine (30%, 6.00 mol) was weighed for later use (denoted as material B). The material A and the material B were delivered by a metering pump to a static mixer preheated to 150°C through pipes, respectively. An outlet of the static mixer was connected to a sealed reaction kettle. A set pressure of a relief valve at an outlet of the sealed reaction kettle was 0.55 MPa. An outlet of a feed pipe was arranged at the bottom of the reaction kettle, and a discharge port pipe was arranged above the liquid level. A feed mass ratio of the material A to the material B was 6.02:1. A suitable feed flow rate was adjusted. A retention time of the materials in the reaction kettle was controlled to be 0.4 h. After the operation was stable, the product was continuously taken out of the discharge port, cooled, and directly let to stand, thereby obtaining clear oil and water phases. Both phases were separated. The resulting oil phase was washed, then distilled under reduced pressure to remove toluene, cooled to 60-65°C, added with 550 g of methanol, warmed for refluxing and dissolution, cooled to around -5°C, crystallized for 3 h, subjected to suction filtration, and washed. The filter cake was dried to obtain 1595.4 g of azoxystrobin product with a content of 98.2% and a yield of 97.1%.

### Example 8

1310 g (4.00 mol) of a compound of Formula I and 544 g (4.48 mol) of 2-cyanophenol were added to 5240 g of toluene, and stirred until a clear solution was obtained for later use (denoted as material A). 1416 g of an aqueous solution of trimethylamine (30%, 7.20 mol) was weighed for later use (denoted as material B). The material A and the material B were delivered, in a feed mass ratio, by a metering pump A and a metering pump B to an inlet of a static mixer through pipes, respectively. A safety relief valve was connected between the metering pump A and the static mixer. The static mixer was preheated to 150°C. An outlet of the static mixer was connected to a retention pipe, and a temperature of the retention pipe was controlled to 170°C. An outlet of the retention pipe was sequentially connected to a cooling coil, a back pressure valve, and a receiving flask. A set pressure of the back pressure valve was 0.95 MPa. A length of the retention pipe was adjusted such that a retention time of the materials in the retention pipe was 12 min. A feed rate ratio of the material A to the material B was set to 5:1. After the operation was stable, the effluent was received, cooled, and directly let to stand, thereby obtaining clear oil and water phases. Both phases were separated. The resulting oil phase was washed, then distilled under reduced pressure to remove toluene, added with methanol, warmed for refluxing and dissolution, cooled to around -5°C, crystallized for 3 h, subjected to suction filtration, and washed. The filter cake was dried to obtain 1583.9 g of azoxystrobin product with a content of 98.0% and a yield of 96.2%.

### Example 9

1310 g (4.00 mol) of a compound of Formula I and 544 g (4.48 mol) of 2-cyanophenol were added to 5240 g of toluene, and stirred until a clear solution was obtained for later use (denoted as material A). 1573 g of an aqueous solution of trimethylamine (30%, 8.00 mol) was weighed for later use (denoted as material B). The material A and the material B were delivered, in a feed mass ratio, by a metering pump A and a metering pump B to an inlet of a static mixer through pipes, respectively. A safety relief valve was connected between the metering pump A and the static mixer. The static mixer was preheated to 160°C for the reaction. An outlet of the static mixer was connected to a retention pipe, and a temperature of the retention pipe was controlled to 160°C. An outlet of the retention pipe was sequentially connected to a cooling coil, a back pressure valve, and a receiving flask. A set pressure of the back pressure valve was 1.1 MPa. A length of the retention pipe was adjusted such that a retention time of the materials in the time delay pipe was 10 min. A feed rate ratio of the material A to the material B was set to 4.51:1. After the operation was stable, the effluent was received, cooled, and directly let to stand, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 1 h, subjected to suction filtration, and washed. The filter cake was dried to obtain 1573.8 g of azoxystrobin product with a content of 97.5% and a yield of 95.1%.

### Example 10

First recycling and reuse of trimethylamine: hydrochloric acid was added to the resulting water phase in Example 4 to adjust the pH to 2-3. Activated carbon accounting for 0.2% of the material by weight was then added, and warmed to 60°C for impurity adsorption for 0.5 h. The filtrate was concentrated at 130°C under normal pressure, pretreated with 53.4 g of a NaOH solution (30%, 0.40 mol), replenished with 4.1 g of an aqueous solution of trimethylamine (30%, 0.021 mol), and then transferred to a reaction kettle. Toluene, 131 g (0.40 mol) of a compound of Formula I, and 51.5 g (0.42 mol) of 2-cyanophenol were added to the reaction kettle. The reaction kettle was closed and warmed to 150°C (with a system pressure of 0.55 MPa), and the temperature was held for reacting for 0.5 h. The temperature was reduced to around 30°C, followed by standing directly, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 158.1 g of azoxystrobin product with a content of 98.5% and a yield of 96.5%.

### Example 11

Hydrochloric acid was added to the resulting water phase in Example 10 to adjust the pH to 2-3. Activated carbon accounting for 0.2% of the water phase by weight was added, warmed to around 60°C, and stirred for impurity adsorption for 0.5 h. The filtrate was concentrated at 130°C under normal pressure, cooled to 50°C, and filtered for salt removal. A small amount of water was added for salt washing. The eluate was mixed with the filtrate, treated with 53.5 g of a NaOH solution (30%, 0.40 mol), replenished with 4.1 g of an aqueous solution trimethylamine (30%, 0.021 mol), and then transferred to a reaction kettle. 500 g of toluene, 131 g (0.40 mol) of a compound of Formula I, and 51.5 g (0.42 mol) of 2-cyanophenol were added to the reaction kettle. The reaction kettle was closed and warmed to 150°C (with a system pressure of 0.55 MPa), and the temperature was held for reacting for 0.5 h. The temperature was reduced to around 30°C, followed by standing directly, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 157.8 g of azoxystrobin product with a content of 98.5% and a yield of 96.3%.

### Example 12

Hydrochloric acid was added to the resulting water phase in Example 11 to adjust the pH to 2-3. Activated carbon accounting for 0.2% of the water phase by weight was added, warmed to around 60°C, and stirred for impurity adsorption for 0.5 h. The filtrate was concentrated at 130°C under normal pressure, cooled to 50°C, and filtered for salt removal. A small amount of water was added for salt washing. The eluate was mixed with the filtrate, replenished with 4.1 g of an aqueous solution trimethylamine (30%, 0.021 mol), treated with 50.7 g of a NaOH solution (30%, 0.38 mol), and then transferred to a reaction kettle. 600 g of toluene, 131 g (0.40 mol) of a compound of Formula I, and 51.5 g (0.42 mol) of 2-cyanophenol were added to the reaction kettle. The reaction kettle was closed and warmed to 150°C (with a system pressure of 0.55 MPa), and the temperature was held for reacting for 0.5 h. The temperature was reduced to around 30°C, followed by standing directly, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 158.3 g of azoxystrobin product with a content of 98.4% and a yield of 96.5%.

### Example 13

Hydrochloric acid was added to the resulting water phase in Example 12 to adjust the pH to 2-3. Activated carbon accounting for 0.2% of the water phase by weight was added, warmed to around 60°C, and stirred for impurity adsorption for 0.5 h. The filtrate was concentrated at 130°C under normal pressure, cooled to 50°C, and filtered for salt removal. A small amount of water was added for salt washing. The eluate was mixed with the filtrate, treated with 56.0 g of a KOH solution (48%, 0.48 mol), and then transferred to a reaction kettle. Toluene, 131 g (0.40 mol) of a compound of Formula I, and 51.5 g (0.42 mol) of 2-cyanophenol were added to the reaction kettle. The reaction kettle was closed and warmed to 150°C (with a system pressure of 0.55 MPa), and the temperature was held for reacting for 0.5 h. The temperature was reduced to around 30°C, followed by standing directly, thereby obtaining clear oil and water phases. Both phases were separated. The resulting oil phase was washed, then distilled under reduced pressure to remove toluene, cooled to 60-65°C, added with methanol, warmed for refluxing and dissolution, cooled to around -5°C, crystallized for 2 h, subjected to suction filtration, and washed. The filter cake was dried to obtain 159.0 g of azoxystrobin product with a content of 98.1% and a yield of 96.7%.

The results of Examples 11-13 showed that the post-treatment of the water phase in the present application was relatively simple, including: adding a small amount of hydrochloric acid (for adjusting the pH to 2-3) to immobilize free trimethylamine (trimethylamine hydrochloride had a high solubility), adding activated carbon for decolorization, concentrating under reduced pressure for salt removal (salt removal was not required for the first reuse; after the second reuse, single salts were removed (NaCl in Examples 11 and 12, and KCl in Example 13); a relatively single type of salts was produced; and the recovered salts could be used as recycled resources), and adding a base to the filtrate to liberate trimethylamine which could be reused directly without separation.

### Example 14

131 g (0.40 mol) of a compound of Formula I, 53.4 g (0.44 mol) of 2-cyanophenol, and 350 g of toluene were added to a sealed reaction kettle. After stirring evenly, 129.8 g of an aqueous solution of trimethylamine (20%, 0.44 mol) was added. The reaction kettle was closed. The temperature was increased to 88°C with stirring. A system pressure was 0.02 MPa. The color of the reaction mixture gradually became lighter in the process of holding the temperature. The temperature was held for reacting for 4 h, and the starting materials were centrally controlled to react completely. The temperature was reduced to 30°C, followed by standing for liquid separation, thereby obtaining clear oil and water phases. Both phases were separated. The resulting reddish-brown oil phase was washed, then distilled under reduced pressure to remove toluene, cooled to 60-65°C, added with methanol, warmed for refluxing and dissolution, cooled to around 0°C, crystallized for 2 h, subjected to suction filtration, and washed. The filter cake was dried to obtain 158.75 g of azoxystrobin product with a content of 98.3% and a yield of 96.7%.

### Example 15

131 g (0.40 mol) of a compound of Formula I, 52.21 g (0.43 mol) of 2-cyanophenol, and 350 g of toluene were added to a sealed reaction kettle. After stirring evenly, 67.97 g of an aqueous solution of trimethylamine (40%, 0.46 mol) was added. The reaction kettle was closed. The temperature was increased to 97°C with stirring. A system pressure was 0.08 MPa. The color of the reaction mixture gradually became lighter in the process of holding the temperature. The temperature was held for reacting for 3.5 h, and the starting materials were centrally controlled to react completely. The temperature was reduced to 35°C, followed by standing for liquid separation, thereby obtaining clear oil and water phases. Both phases were separated. The resulting reddish-brown oil phase was washed, then distilled under reduced pressure to remove toluene, cooled to 60-65°C, added with methanol, warmed for refluxing and dissolution, cooled to around -5°C, crystallized for 1.5 h, subjected to suction filtration, and washed. The filter cake was dried to obtain 159.22 g of azoxystrobin product with a content of 98.2% and a yield of 96.9%.

### Example 16

131 g (0.40 mol) of a compound of Formula I, 55.91 g (0.46 mol) of 2-cyanophenol, and 398 g of toluene were added to a sealed reaction kettle. After stirring evenly, 102.45 g of an aqueous solution of trimethylamine (30%, 0.52 mol) was added. The reaction kettle was closed. The temperature was increased to 120°C with stirring. A system pressure was 0.19 MPa. The color of the reaction mixture gradually became lighter in the process of holding the temperature. The temperature was held for reacting for 1.3 h, and the starting materials were centrally controlled to react completely. The temperature was reduced to 35°C, followed by standing for liquid separation, thereby obtaining clear oil and water phases. Both phases were separated. The resulting reddish-brown oil phase was washed, then distilled under reduced pressure to remove toluene, cooled to 60-65°C, added with methanol, warmed for refluxing and dissolution, cooled to around 0°C, crystallized for 2 h, subjected to suction filtration, and washed. The filter cake was dried to obtain 158.56 g of azoxystrobin product with a content of 98.0% and a yield of 96.3%.

### Comparative Example 1: using liquid caustic soda as an acid scavenger

450 g of toluene, 120 g (0.370 mol, 99%) of a compound of Formula I, 49 g (0.408 mol, 99%) of 2-cyanophenol, 5.31 g of an aqueous solution of trimethylamine (0.0297 mol, 33%), and 53.55 g (0.428 mol, 32%) of liquid caustic soda were added to a 1000 mL four-necked flask. The temperature was slowly increased to 80°C. The color of the reaction mixture became deeper in the process of holding the temperature, and the temperature was held for 10 h. After the completion of the reaction, phases were separated, thereby obtaining 597.2 g of a dark red toluene liquid, with an external standard azoxystrobin content of 22.73% and a conversion rate of 90.7%.

Comparative Example 1 showed that when liquid caustic soda was used as the acid scavenger, the conversion rate of the product decreased. As speculated by the inventors, it was probably because liquid caustic soda has extremely strong alkalinity, resulting in byproducts. The presence of the base in a large amount at a high temperature leads to the hydrolysis of ester groups of the compound of Formula I and azoxystrobin and the substitution of Cl on the compound of Formula I with hydroxyl.

### Comparative Example 2: using potassium carbonate as an acid scavenger

300 g of toluene, 162 g (0.500 mol, 99%) of a compound of Formula I (methyl (E)-2-[2-(6-chloropyrimidin-4-yloxy)phenyl]-3-methoxyacrylate), 66.15 g (0.550 mol, 99%) of 2-cyanophenol, and 55.8 g (0.4 mol, 99%) of potassium carbonate were sequentially added to a 1000 mL reaction flask. The reaction mixture was difficult to stir due to a high solid content, and thus was replenished with 150 g of toluene to enable smooth stirring. 7.15 g of an aqueous solution of trimethylamine (0.04 mol, 33%) was added. The reaction mixture was stirred under normal pressure and warmed to 80°C, releasing an alkaline gas from a condenser. The alkaline gas caused the reaction mixture to foam, leading to volume expansion. The temperature of the reaction mixture was held for 10 h. After the completion of the reaction, 200 g of water was added for layering, thereby obtaining 650.03 g of a solution of azoxystrobin in toluene with a content of 29.85% and a conversion rate of 96.2%.

In Comparative Example 2, trimethylamine in the water phase could be recycled and reused. However, due to the water phase also contained mixed salts of chloride, bicarbonate and carbonate, the post-treatment of the water phase was relatively complicated, including: using nitrogen to blow away trimethylamine and adding water (or methanol) for absorption (trimethylamine had a high solubility in water, 20 g/100 g (30°C), and a large amount of N₂ was needed for guaranteeing the recovery rate of trimethylamine). Saltwater left after the recovery of trimethylamine needed to be added with an acid (usually hydrochloric acid) to remove excess carbonate and bicarbonate, and then was decolorized. The filtrate was concentrated for salt removal. In Comparative Example 2, the reaction mixture needed to be added with water for dissolving the salt until a clear liquid was obtained before liquid separation, and a large amount of hydrochloric acid was needed for converting the water phase obtained after liquid separation into chloride, thus greatly increasing the post-treatment cost. The recovery of trimethylamine also required a large amount of water. That is, this comparative example required a use of a large amount of water, leading to wasting of water resources.

In Comparative Example 2, solid potassium carbonate was fed. The operations were complicated, and a long reaction time (10 h) was needed. In industrial production, a manhole needed to be formed or a solid feeding bin needed to be provided. Theoretically, 1 t of product was accompanied with 54.6 kg of byproduct CO₂. The reaction mixture imposed a high requirement on the stirring device. The system might not be sealed, and the reaction would occur under normal pressure.

### Comparative Example 3: using triethylamine as an acid scavenger

230 g of toluene, 81 g (0.25 mol, 99%) of a compound of Formula I (methyl (E)-2-[2-(6-chloropyrimidin-4-yloxy)phenyl]-3-methoxyacrylate), 33.06 g (0.275 mol, 99%) of 2-cyanophenol, and 20.44 g (0.2 mol, 99%) of triethylamine were sequentially added to a 500 mL reaction flask. The temperature was increased to 90°C with stirring. The temperature of the reaction mixture was held for 10 h. 100 g of water was added for layering, thereby obtaining 356.78 g of a solution of azoxystrobin in toluene with a content of 6.47% and a yield of 22.89%.

In Comparative Example 3, although having a similar structure to trimethylamine, triethylamine used as the acid scavenger failed to make the reaction proceed effectively with a low conversion rate.

### Comparative Example 4: using a trimethylamine-methanol solution as an acid scavenger

230 g of toluene, 81 g (0.25 mol, 99%) of a compound of Formula I (methyl (E)-2-[2-(6-chloropyrimidin-4-yloxy)phenyl]-3-methoxyacrylate), 33.06 g (0.275 mol, 99%) of 2-cyanophenol, and 49.2 g (0.275 mol, 33%) of a trimethylamine-methanol solution were sequentially added to a 500 mL reaction flask. The temperature was increased with stirring, and a large amount of alkaline gas was released in the warming process. After a reflux reaction for 10 h, the conversion of the starting materials was stopped. 200 g of water was added, and stirred and let to stand for phase seperation. As a result, 335.1 g of oil phase was obtained with a content of 23.56% and a conversion rate of 78.29%.

In Comparative Example 4, with the trimethylamine-methanol solution as the acid scavenger, while the reaction system also contained trimethylamine, the conversion efficiency of the reaction was low. Comparative Example 5: using an aqueous solution of potassium carbonate as an acid scavenger

450 g of toluene, 162 g (0.500 mol, 99%) of a compound of Formula I (methyl (E)-2-[2-(6-chloropyrimidin-4-yloxy)phenyl]-3-methoxyacrylate), 66.15 g (0.550 mol, 99%) of 2-cyanophenol, and 55.8 g (0.4 mol, 99%) of potassium carbonate were sequentially added to a 1000 mL reaction flask, and 7.15 g of an aqueous solution trimethylamine (0.04 mol, 33%) and 55 g of water were added thereto, forming a water-oil system. The reaction mixture was stirred under normal pressure and warmed to 80°C, releasing an alkaline gas from a condenser. Foaming was observed in the reaction mixture, accompanied by the gradual precipitation of a significant quantity of salt. The temperature of the reaction mixture was held for 9 h. After the completion of the reaction, 150 g of water was added for dissolving the salt and layering, thereby obtaining 649.87 g of a solution of azoxystrobin in toluene with a content of 28.83% and a conversion rate of 92.89%.

Compared with Comparative Example 2, a certain amount of water was added to dissolve potassium carbonate at the early stage of the reaction, resulting in reduced reaction conversion rate. Moreover, with the reaction proceeding, byproducts potassium chloride and potassium bicarbonate were precipitated, forming a gas-liquid-solid triphasic system. After the completion of the reaction, water still needed to be added for dissolving salts. The final saltwater weighed about 260 g, which was increased by approximately 68% as compared to a weight of a feed batch of the aqueous solution of trimethylamine (in Example 2, the saltwater weighed about 124 g).

Furthermore, Comparative Example 4 was the same as Comparative Example 2, both requiring feeding of solid potassium carbonate. The operations were complicated, and the reaction time was long. In industrial production, the manhole needed to be formed or the solid feeding bin needed to be provided. Theoretically, 1 t of a product was accompanied with 54.6 kg of a byproduct CO₂. The reaction mixture imposed a high requirement on the stirring device. The system might not be sealed, and the reaction would occur under normal pressure.

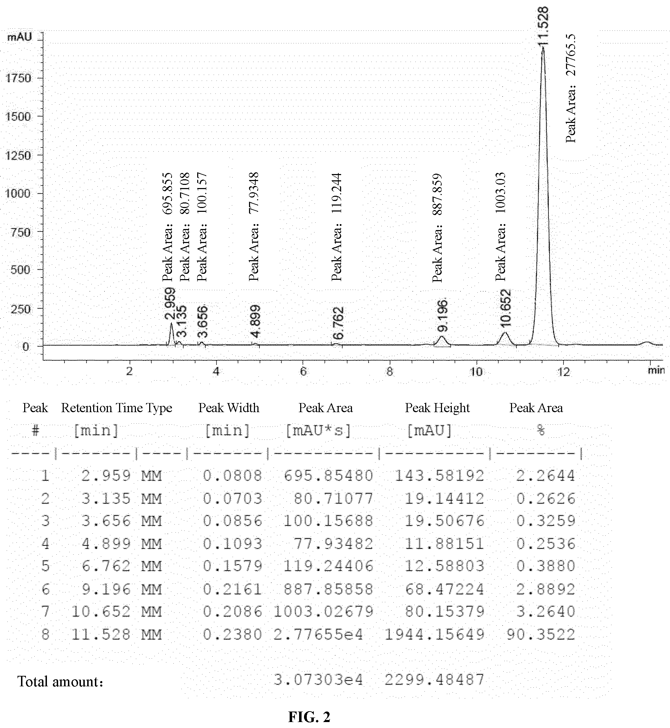

Comparative Examples 1 to 5 showed that with potassium carbonate as the acid scavenger and trimethylamine as the catalyst in Comparative Example 2, the yield of the product was relatively high, but there were many problems: Comparative Example 2 (conventional process) involved a solid-liquid heterogeneous reaction, and the color of the materials dissolved with stirring was black brown, i.e., some colored impurity byproducts were generated (see A in FIG. 1). In the warming or temperature holding process, heterogeneous materials were prone to attaching on the wall while stirring (see C and E in FIG. 1). In practical industrial production, solid-phase materials would easily wear reactors, resulting in increased maintenance costs. After the completion of the reaction, a large amount of inorganic salt was deposited at the bottom (see G in FIG. 1), and water needed to be added for liquid separation. After the completion of the reaction, the color of the organic phase was still deep (see G in FIG. 1). After water was added for layering, HPLC analysis was performed on the organic phase, with results shown in FIG. 2. The results showed that a high content of three kinds of impurities (with respective impurity peaks at 2.959 min, 9.196 min, and 10.652 min) was generated, and a proportion of impurities was approximately 8.4%. The crystallization step should be operated more strictly, avoiding impurity entrainment from affecting the quality and increasing the cost.

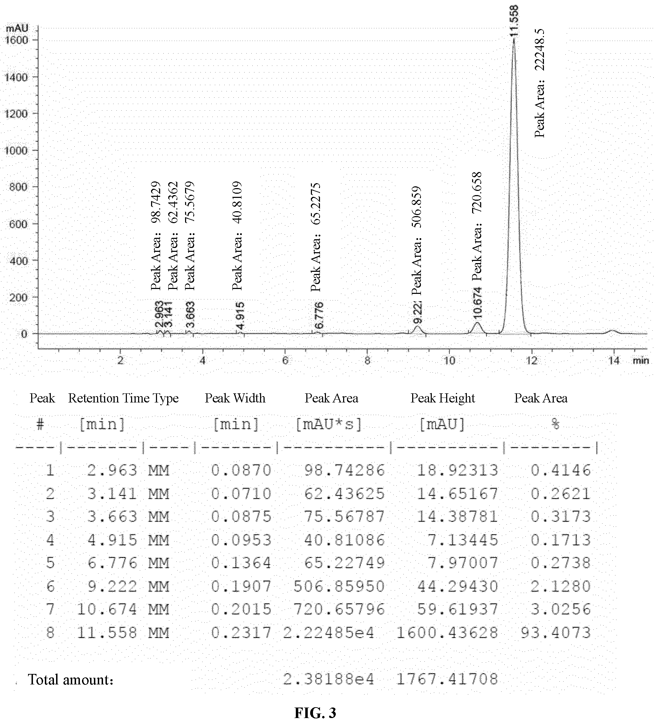

According to the present application, trimethylamine, especially in an aqueous solution, is fed as the acid scavenger for synthesis of azoxystrobin. It is not required to add an additional acid acceptor or convert 2-cyanophenol into 2-cyanophenol salts. There is also no need to use solid potassium carbonate or sodium carbonate. For example in Example 3, the material reaction system was an oil-water reaction system, and the organic phase was reddish brown (which was obviously lighter than the color in FIG. 1A), indicating fewer colored impurity byproducts (see B in FIG. 1), and easier stirring operation in the warming or temperature holding process with stirring (see D and F in FIG. 1) was enabled. After the completion of the reaction, clear oil and water phases could be obtained just by standing. The color of the organic phase did not deepen, indicating fewer colored impurities generated in the reaction process (see H in FIG. 1). The organic phase was subjected to HPLC analysis, with results shown in FIG. 3. The results showed that there were only two kinds of impurities with high proportions of impurity peaks (with respective impurity peaks at 9.222 min and 10.674 min). A proportion of impurities was only 5.1% (obviously less than the proportion of impurities in Comparative Example 2), and a proportion of effective component was 93.4%. The content of the effective component was higher. The post-treatment cost was reduced. Direct liquid separation can be carried out without an addition of water, thereby greatly improving the efficiency and avoiding wasting of water resources and post-treatment of a large amount of mixed salts in conventional techniques.

According to the present application, the use of an aqueous solution of trimethylamine can effectively reduce the generation of colored impurities, and also can reduce the cost and difficulty of purification while increasing the reaction yield. Also, easy hydrolysis of methyl (E)-2-(2-((6-chloropyrimidin-4-yl)oxy)phenyl)-3-methoxyacrylate when a large amount of water is present in the sodium/potassium carbonate system is prevented.

Furthermore, the inventors have also founded that when a salt of 2-cyanophenol is used, trimethylamine would overflow in a large amount, which not only affects the environment, but also increases the treatment difficulties.

The inventors have also studied the stability of 2-cyanophenol, and have found that it would be polymerized gradually at a temperature of 110°C or above, and after holding the temperature for 0.5 h, the clear solution of 2-cyanophenol has significantly become turbid. Besides, with increasing temperature and prolonging time, the extent of polymerization will increase. 2-cyanophenol would be cured after cooling. Therefore, by improving the reaction efficiency, the generation of the polymerization byproducts of 2-cyanophenol can be effectively reduced.

The reaction temperature of 50°C to 170°C, the reaction pressure of from normal pressure to 1.1 Mpa, and the reaction time of 5 min to 360 min are used in the present application. The reaction conditions are mild, providing high efficiency, and the reaction time is short. The present application proposes a method of synthesizing azoxystrobin with trimethylamine as an acid scavenger under normal pressure or a micro-positive pressure, in which the reaction may be intermittent or continuous.

In a kettle-based intermittent or continuous reaction, the reaction endpoint can be reached by holding the temperature for 30 min at 150°C and under a pressure of 0.55 MPa.

In the kettle-based intermittent reaction, the reaction endpoint can be reached by holding the temperature for 4 h to 5 h at 85°C to 90°C and under normal pressure.

The reaction time is relatively long, which is about 4 h to 5 h, under normal pressure and at the reflux temperature (e.g., Examples 1 to 3). However, where trimethylamine is used as the acid scavenger instead of solid sodium carbonate or potassium carbonate, high reaction yield and purity can still be achieved.

In the present application, the reaction may be a kettle-based intermittent reaction, a kettle-based continuous reaction, or a pipe-based continuous reaction. In the pipe-based continuous reaction, efficient synthesis can be achieved in extremely short time, thereby improving the production efficiency.

When the pipe-based continuous reaction is adopted, at a reaction temperature of 150°C, the reaction endpoint can be reached after staying for about 10 min. The pipe-based continuous reaction equipment comprises a feeding pump, a safety relief valve, a static mixer, a retention pipe, a back pressure valve, a receiving device, and a heat exchanger.

The same post-treatment solution is adopted in the above-mentioned kettle-based intermittent reaction, kettle-based normal-pressure continuous reaction, and pipe-based continuous reaction. Phases are separated after the completion of the reaction. The pH of the water phase is adjusted to 2-8. An alkaline substance is added after concentration. The alkaline substance is NaOH or KOH and is added in an amount 0.95 to 1.2 times the molar weight of methyl (E)-2-(2-((6-chloropyrimidin-4-yl)oxy)phenyl)-3-methoxyacrylate, and can be recycled and reused for next batch. For continuous reuse, NaCl (or KCl) is separated by filtration after concentration. The saltwater left after the separation of NaCl (or KCl) is added with a base for continuous reuse. The oil phase is washed, desolvated, refined, separated, and dried.

According to the present application, the use of trimethylamine as the acid scavenger can effectively avoid cyano-polymerization of 2-cyanophenol. Moreover, increasing the usage amount of trimethylamine can significantly accelerate the reaction, avoiding the hydrolysis or alcoholysis of the starting material methyl (E)-2-(2-((6-chloropyrimidin-4-yl)oxy)phenyl)-3-methoxyacrylate, as well as the occurrence of side reactions involving 2-cyanophenol during the heating process. The problems of easy foaming and overflow in the reaction process in the prior art are solved. Also, difficulties in post-treatment of the water phase are reduced (the use of sodium carbonate in the conventional process results in generation of mixed salts of chloride, bicarbonate, and carbonate, and therefore, a large amount of acid needs to be added to neutralize them into chloride after dissolution and liquid separation, thereby increasing not only costs but also carbon emissions and environmental pollution), and wear of reactors by solid materials is avoided. According to the present application, an aqueous solution of trimethylamine is used as the acid scavenger, eliminating the need for potassium carbonate or sodium carbonate, as well as the conversion of 2-cyanophenol to a salt of 2-cyanophenol. The material reaction system is an oil-water reaction system, which is conducive to molecule or ion diffusion and improvement of the reaction efficiency. Moreover, trimethylamine can be recycled and reused, avoiding complicated treatment of mixed salts in post-treatment. Liquid separation can be realized without an addition of additional water, avoiding wasting of water resources. The reaction is efficient and the time is shortened.

According to the present application, there is no need for a catalyst so that the reaction system can be simpler. The acid scavenger used in the present application can be recycled and reused, avoiding the generation of industrial waste salts and greenhouse gas CO₂ due to the use of sodium carbonate or potassium carbonate as in the conventional techniques, and allowing for cleaner production and lower costs. The present application has the advantages of absence of solids in the reaction mixture, convenient operations, continuous reaction achievable with conventional equipments, improved production efficiency, reduced manpower and equipment inputs, and low costs. The present application addresses the problems of the solid-liquid heterogeneous reaction system, achieves efficient mass transfer and heat transfer in the reaction process, higher production efficiency, and cleaner production. The present application has no gas generated, thereby fundamentally avoiding the risk of overflow and realizing the intrinsic safety of the production process.

Finally, it should be noted that the foregoing examples are only used to illustrate the technical solutions of the present application, and are not intended to limit the present application. Although the present application is described in detail with reference to the foregoing examples, those of ordinary skill in the art should understand that they can still modify the technical solutions described in the foregoing examples, or make equivalent substitutions to some technical features therein. These modifications or substitutions do not make the essence of the corresponding technical solutions depart from the spirit and scope of the technical solutions in the examples of the present application.

### Industrial Applicability

The present application discloses a synthesis method for azoxystrobin, comprising a step of: reacting a compound of Formula I with 2-cyanophenol in a water-oil system of an organic solvent and an aqueous solution of trimethylamine to obtain azoxystrobin, wherein a molar ratio of the compound of Formula I to trimethylamine is 1:(0.96-2). The synthesis method of the present application has advantages of high reaction efficiency, no CO₂ generation, avoidance of the risk of entrained overflow, reduced secondary reactions, reduced solid waste emissions, and environmental friendliness. Use of solid sodium carbonate or potassium carbonate is avoided and the material reaction system is an oil-water reaction system. The reaction efficiency is significantly improved and the yield can reach 95% or above. Both efficiency (timeliness) and yield can be taken into account, and continuous reactions can be realized easily, providing technical support for intelligent, unmanned production. Moreover, trimethylamine can be recycled, reducing the consumption of one starting material. Accordingly, complicated treatment of mixed salts and wastewater thereof can be avoided, thereby significantly reducing energy consumption.

## Claims

1. A synthesis method for azoxystrobin, comprising a step of: reacting a compound of Formula I with 2-cyanophenol in a water-oil system of an organic solvent and an aqueous solution of trimethylamine to obtain azoxystrobin, 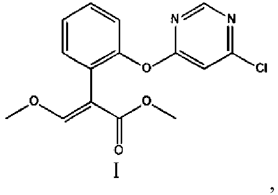 wherein a molar ratio of the compound of Formula I to trimethylamine is 1:(0.96-2).

2. The synthesis method according to claim 1, wherein the molar ratio of the compound of Formula I to trimethylamine is 1:(1-2), optionally 1:(1.06-2), optionally 1:(1.1-2), optionally 1:(1.06-1.8), and optionally 1:(1.1-1.8);
and/or a molar ratio of the compound of Formula I to 2-cyanophenol is 1:(1-5), optionally 1:(1-1.5), and optionally 1:(1-1.2);
and/or a mass fraction of trimethylamine in the aqueous solution of trimethylamine is 20% to 40%, optionally 25% to 40%, optionally 20% to 30%, optionally 25% to 30%, and optionally 30% to 40%;
and/or a molar ratio of the compound of Formula I to water is 1:(4.728-23.64), optionally 1:(4.925-23.64), optionally 1:(5.2205-23.64), optionally 1:(5.66-23.64), optionally 1:(7.65-23.64), optionally 1:(5.66-15.29), and optionally 1:(7.65-15.29);
and/or a molar fraction of water in the water-oil system is 30mol% to 60mol%, optionally 3 1mol% to 60mol%, optionally 31mol% to 54mol%, optionally 31mol% to 53mol%, and optionally 35mol% to 53mol%;
and/or no additional sodium carbonate or potassium carbonate needs to be added.

3. The synthesis method according to claim 1, wherein an addition amount of the organic solvent is at least an amount for dissolving the compound of Formula I and 2-cyanophenol;
and/or a weight ratio of the compound of Formula I to the organic solvent is 1:(2-8), optionally 1:(2-5.7), optionally 1:(2-4), optionally 1:(2.3-5.7), and optionally 1:(2.3-4);
and/or the organic solvent comprises toluene;
and/or the reaction occurs in the water-oil system composed of the organic solvent and the aqueous solution of trimethylamine.

4. The synthesis method according to claim 1, wherein a reaction temperature is 50°C to 170°C, optionally 88°C to 170°C, optionally 110°C to 170°C, optionally 140°C to 170°C, optionally 140°C to 160°C, optionally 80°C to 150°C, and optionally 80°C to a reflux temperature;
and/or a reaction pressure is greater than normal pressure, optionally 0.12 MPa to 1.2 MPa, optionally 0.29 MPa to 1.2 MPa, optionally 0.5 MPa to 1.2 MPa, and optionally 0.55 MPa to 1.1 MPa;
and/or a reaction time is 5 min to 360 min, optionally 15 min to 360 min, optionally 30 min to 360 min, optionally 5 min to 60 min, optionally 30 min to 60 min, and optionally 5 min to 30 min.

5. The synthesis method according to claim 1, wherein the reaction occurs in a pipe-based continuous reaction equipment; a pipe temperature is 110°C to 170°C, optionally 140°C to 160°C; optionally, a reaction pressure is 0.55 MPa to 1.1 MPa, optionally 0.5 MPa to 0.7 MPa;
optionally, in the pipe-based continuous reaction, the compound of Formula I, 2-cyanophenol, and the organic solvent are used as a material A, and the aqueous solution of trimethylamine is used as a material B; and a mass ratio of the material A to the material B is (4-7):1 such that the molar ratio of the compound of Formula I to trimethylamine in the system is maintained at 1:(1-2), optionally 1:(1.06-1.8), and optionally 1:(1.1-1.8);
or, a kettle-based intermittent reaction or a kettle-based continuous reaction is employed with a reaction temperature of 85°C to 150°C, optionally a reaction temperature of 85°C to 95°C; optionally, a reaction pressure is normal pressure to 1.1 MPa; optionally, the reaction pressure is greater than normal pressure, and ≤1.1 MPa; and optionally, the reaction pressure is 0.12 MPa to 1.1 MPa.

6. The synthesis method according to claim 5, wherein the pipe-based continuous reaction equipment comprises a feeding pump, a safety relief valve, a static mixer, a retention pipe, a back pressure valve, a receiving device, and a heat exchanger.

7. The synthesis method according to any one of claims 1 to 6, wherein after completion of the reaction, post-treatment is carried out to obtain azoxystrobin, which comprises steps of: direct liquid separation of materials after the completion of the reaction, oil phase desolvation, and purification.

8. The synthesis method according to claim 7, wherein the purification comprises steps of: redissolution, crystallization, suction filtration, washing and drying, and optionally, the redissolution occurs in methanol.

9. The synthesis method according to claim 7, wherein trimethylamine is recovered from a water phase after liquid separation by a process comprising steps of: adjusting a pH of the water phase to 2-8, concentrating, and then adding a base to obtain a solution containing trimethylamine.

10. The synthesis method according to claim 9, wherein in the trimethylamine recovery, an addition amount of the base is 0.95 to 1.2 times or 1 to 1.2 times a molar weight of the compound of Formula I;
and/or in the trimethylamine recovery, the base is an alkali metal hydroxide;
and/or the base is used for liberating trimethylamine.
